(19) Europäisches Patentamt
European Patent Office
Office européen des brevets

(11) Veröffentlichungsnummer: **0 464 487 A1**

# (12) EUROPÄISCHE PATENTANMELDUNG

(21) Anmeldenummer: **91110141.8**

(22) Anmeldetag: **20.06.91**

(51) Int. Cl.5: **C07C 233/09**, C07B 57/00, C08F 20/54, C07D 319/06, B01J 20/32

(30) Priorität: **03.07.90 DE 4021106**

(43) Veröffentlichungstag der Anmeldung:
**08.01.92 Patentblatt 92/02**

(84) Benannte Vertragsstaaten:
**AT BE CH DE DK ES FR GB GR IT LI LU NL SE**

(71) Anmelder: **BAYER AG**

**W-5090 Leverkusen 1 Bayerwerk(DE)**

(72) Erfinder: **Grosser, Rolf, Dr.**
**Gellertstrasse 9**
**W-5090 Leverkusen(DE)**
Erfinder: **Lange, Walter, Dr.**
**Auerstrasse 7**
**W-5000 Köln 60(DE)**
Erfinder: **Bömer, Bruno, Dr.**
**Max-Planck-Strasse 53**
**W-5060 Bergisch Gladbach(DE)**
Erfinder: **Arlt, Dieter, Prof. Dr.**
**Rybniker Strasse 2**
**W-5000 Köln 80(DE)**
Erfinder: **Gassen, Karl-Rudolf, Dr.**
**Auenweg 6a**
**W-5068 Odenthal(DE)**

(54) **Optische aktive Alpha-Fluoracrylsäureamide, ihre Herstellung, ihre Polymerisation zu optisch aktiven Polymeren und deren Verwendung zur Trennung von Racematen.**

(57) Die Erfindung betrifft neue optisch tive $\alpha$-Fluoracrylsäureamide der allgemeinen Formel (I)

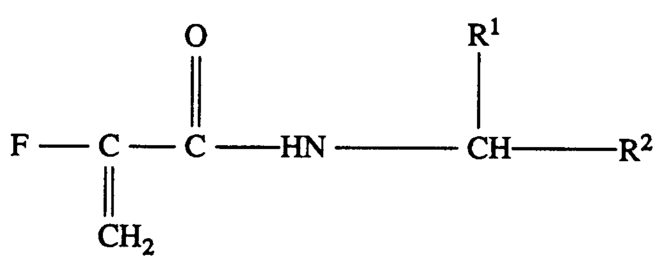

(I)

in welcher $R_1$ und $R_2$ die in der Beschreibung angegebene Bedeutung haben, ein Verfahren zu ihrer Herstellung, ihre Polymerisation zu optisch aktiven Polymeren und die Verwendung dieser optisch aktiven Polymere als Adsorbentien für die chromatographische Trennung von Racematen in ihre Enantiomere.

EP 0 464 487 A1

Die Erfindung betrifft neue optisch aktive α-Fluoracrylsäureamide, ein Verfahren zu ihrer Herstellung, ihre Polymerisation zu optisch aktiven Polymeren und die Verwendung dieser optisch aktiven Polymere als Adsorbentien für die chromatographische Trennung von Racematen in ihre Enantiomere.

In den letzten Jahren gewinnt die Trennung von Wirkstoff-Racematen zunehmend an Bedeutung, weil sich gezeigt hat, daß die Enantiomere eines Wirkstoff-Racemats sich häufig in ihren biologischen Wirkungen und Nebenwirkungen unterscheiden.

Neben den klassischen Verfahren zur Racematspaltung hat sich in letzter Zeit die chromatographische Racemattrennung besonders bewährt. Neben Naturstoff-Derivaten z.B. auf Cellulose-Basis sind vermehrt synthetische optisch aktive polymere (Meth)acrylamide als Adsorbentien eingesetzt worden (vgl. G. Blaschke, Chromatogr. Sci. 1988, 40, 179 bis 198).

Bei der Anwendung der bekannten Methoden hat sich jedoch gezeigt, daß diese entweder nur eine unzureichende Wirkung aufweisen oder nur eine auf bestimmte Racemate begrenzte Wirksamkeit aufweisen.

Es wurde nun überraschend gefunden, daß Polymere aus optisch aktiven α-Fluoracrylsäureamiden Adsorbentien mit sehr guten Racemattrenneigenschaften darstellen.

Überraschenderweise führt der Ersatz des Wasserstoffatoms bzw. der Methylgruppe in den (Meth)-acrylamiden durch das stark elektronegative Fluoratom nicht zu einem Verlust an Trennwirkung, wie man aufgrund des gegensätzlichen elektronischen Effekts und der Nähe des Fluoratoms zur Amidgruppe, die ja über Wasserstoffbrücken mit den Enantiomeren des zu trennenden Racemats wechselwirken muß, annehmen würde.

Es wurde gefunden, daß trotz der drastisch veränderten sterischen und elektronischen Umgebung der für die Trennung relevanten Amid-Gruppe die erfindungsgemäßen α-Fluoracrylamid-Polymere gute Trennwirkungen für viele Wirkstoff-Racemate aus den verschiedensten Substanzklassen zeigen, wobei in vielen Fällen insbesondere die Trennselektivität für eine Reihe der an den entsprechenden (Meth)acrylamid-Derivaten unbefriedigend trennbaren Racemate deutlich verbessert wird.

Dies ist überraschend, da man den anderen durch das Fluoratom erwarteten Effekt, nämlich ein lipophileres unpolares Polymer zu erhalten, durchaus beobachtet. Auch die Monomere sind viel unpolarer und besser in Solventien wie Petrolether und Toluol löslich, als die entsprechenden (Meth)acrylamide.

Die Erfindung betrifft optisch aktive α-Fluoracrylsäureamide der Formel (I)

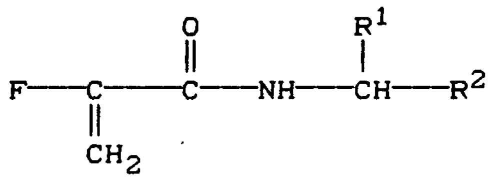

(I)

in welcher

$R^1$ für einen Phenyl-, Naphthyl-, Pyridyl- oder $C_3$-$C_{10}$-Cycloalkylrest steht, der gegebenenfalls substituiert ist durch Halogen, $C_1$-$C_4$-Alkoxy, $NR^3COR^4$, Dioxyalkylen mit 1 bis 2 C-Atomen, Alkyl oder Cycloalkyl mit jeweils bis zu 10 C-Atomen,
wobei

$R^3$ für Wasserstoff oder $C_1$-$C_4$-Alkyl steht und

$R^4$ für Alkyl oder Alkoxy mit jeweils 1 bis 6 C-Atomen, für Phenyl oder Benzoxy steht,

$R^2$ für Alkyl oder Cycloalkyl mit jeweils bis zu 8 C-Atomen, gegebenenfalls substituiert durch Halogen oder durch $C_1$-$C_4$-Alkoxy steht,
oder

$R^1$ und $R^2$ gemeinsam für einen cyclischen Rest aus der Gruppe Terpenyl, 8-Alkyl($C_1$-$C_4$)-menthyl, 8-Aryl($C_6$-$C_{10}$)-menthyl, Tetrahydropyryl, 2,2-Dialkyl($C_1$-$C_4$)-4-aryl($C_6$-$C_{10}$)-1,3-dioxan-5-yl, 2,2-Dialkyl($C_1$-$C_4$)-4-cycloalkyl($C_3$-$C_8$)-1,3-dioxan-5-yl, 2-Spiro-cycloalkyl($C_3$-$C_8$)-aryl-($C_6$-$C_{10}$)-1,3-dioxan-5-yl, 2-Spiro-cycloalkyl-($C_3$-$C_8$)-4-cycloalkyl($C_3$-$C_8$)-1,3-dioxan-5-yl, $C_1$-$C_4$-alkylsubstituierter $C_3$-$C_8$-Cycloalkylrest steht.

Von besonderem Interesse sind Verbindungen der allgemeinen Formel (I), in welcher

$R^1$ für Naphthyl, Pyridyl oder Phenyl steht, welches gegebenenfalls ein- oder zweifach substituiert ist durch gleiche oder verschiedene Substituenten aus der Gruppe Fluor, Chlor, Alkyl mit 1 bis 4 C-Atomen, Alkoxy mit 1 bis 4 C-Atomen, Cycloalkyl mit 3 bis 6 C-Atomen, Methylendioxy, Ethylendioxy, Acetamido, Benzamido, Carbomethoxyamino, Carboethoxyamino, Carbobenzoxyamino oder für einen Cycloalkylrest mit 3 bis 7 C-Atomen oder für einen Decahydronaphthylrest steht,

$R^2$ für Alkyl oder Cycloalkyl mit jeweils bis zu 8 C-Atomen steht, wobei der Alkylrest gegebenenfalls durch Fluor, Chlor oder Alkoxy mit 1 bis 2 C-Atomen substituiert ist, oder

$R^1$ und $R^2$ gemeinsam für einen Rest stehen aus der Gruppe Menthyl, Neomenthyl, Isomenthyl, Neoisomenthyl, Carvomenthyl, Bornyl, Fenchyl, Pinanyl, Isopinocampheyl, 8-Methylmenthyl, 8-Ethyl-menthyl, 8-n-Propyl-menthyl, 8-i-Propylmenthyl, 8-n-Butylmenthyl, 8-i-Butylmenthyl, 8-s-Butylmenthyl, 8-t-Butylmenthyl, 8-Phenylmenthyl, 8-Naphthylmenthyl, Tetrahydropyryl, 2,2-Dimethyl-4-phenyl-1,3-dioxan-5-yl, 2,2-Dimethyl-4-naphthyl-1,3-dioxan-5-yl, 2,2-Diethyl-4-phenyl-1,3-dioxan-5-yl, 2,2-Diethyl-4-naphthyl-1,3-dioxan-5-yl, 2,2-Dipropyl-4-phenyl-1,3-dioxan-5-yl, 2,2-Dipropyl-4-naphthyl-1,3-dioxan-5-yl, 2,2-Dibutyl-4-phenyl-1,3-dioxan-5-yl, 2,2-Dibutyl-4-naphthyl-1,3-dioxan-5-yl analog mit 2,2-Dibutyl-4-cyclohexyl-1,3-dioxan-5-yl, Spirocyclopentyl-4,2'-4-phenyl-1,3-dioxan-5-yl, Spiro-cyclopentyl-4,2'-4-naphthyl-1,3-dioxan-5-yl, Spiro-cyclopentyl-4,2'-4-cyclohexyl-1,3-dioxan-5-yl, Spiro-cyclohexyl-5,2'-4-phenyl-1,3-dioxan-5-yl, Spiro-cyclohexyl-5,2'-4-naphthyl-1,3-dioxan-5-yl, Spiro-cyclohexyl-5,2'-4-cyclohexyl-1,3-dioxan-5-yl, 2-Methyl-cyclopentyl, 2-Ethyl-cyclopentyl, 2-Propyl-cyclopentyl, 2-i-Propyl-cyclopentyl, 2-n-Butyl-cyclopentyl, 2-i-Butyl-cyclopentyl, 2-s-Butyl-cyclopentyl, 2-t-Butyl-cyclopentyl, 2-Methyl-cyclohexyl, 2-Ethyl-cyclohexyl, 2-Propyl-cyclohexyl, 2-i-Propyl-cyclohexyl, 2-n-Butyl-cyclohexyl, 2-i-Butyl-cyclohexyl, 2-s-Butyl-cyclohexyl, 2-t-Butyl-cyclohexyl.

Von ganz besonderem Interesse sind Verbindungen der allgemeinen Formel (I), in welcher

$R^1$ für Phenyl steht, welches gegebenenfalls einfach substituiert ist durch Fluor, Chlor, Methyl, Methoxy, Nitro, Acetamido, Benzamido, Carbomethoxyamino, Carboethoxyamino, Ethyl, Propyl oder Butyl oder für 1-Naphthyl, 2-Naphthyl, 2-Pyridyl, 3-Pyridyl, 4-Pyridyl, Decahydronaphthyl oder Cycloalkyl mit 3 bis 6 C-Atomen steht,

$R^2$ für Alkyl mit 1 bis 4 C-Atomen, welches gegebenenfalls durch Fluor, Chlor, Methoxy oder Ethoxy substituiert ist oder für Cyclopentyl oder Cyclohexyl steht oder

$R^1$ und $R^2$ gemeinsam für einen Rest stehen aus der Gruppe Menthyl, Neomenthyl, Isomenthyl, Neoisomenthyl, Carvomenthyl, Bornyl, Fenchyl, Pinanyl, Isopinocampheyl, 8-Methylmenthyl, 8-Phenylmenthyl, Tetrahydropyryl, 2,2-Dimethyl-4-phenyl-1,3-dioxan-5-yl, 2,2-Diethyl-4-phenyl-1,3-dioxan-5-yl, 2,2-Diethyl-4-phenyl-1,3-dioxan-5-yl, 2,2-Dipropyl-4-phenyl-1,3-dioxan-5-yl, 2,2-Dibutyl-4-phenyl-1,3-dioxan-5-yl, 2,2-Dimethyl-4-cyclohexyl-1,3-dioxan-5-yl, 2,2-Diethyl-4-cyclohexyl-1,3-dioxan-5-yl, 2,2-Dipropyl-4-cyclohexyl-1,3-dioxan-5-yl, 2,2-Dibutyl-4-cyclohexyl-1,3-dioxan-5-yl, Spiro-cyclopentyl-4,2'-4-phenyl-1,3-dioxan-5-yl, Spiro-cyclopentyl-4,2'-4-cyclohexyl-1,3-dioxan-5-yl, Spiro-cyclohexyl-5,2'-4-phenyl-1,3-dioxan-5-yl, Spiro-cyclohexyl-5,2'-4-cyclohexyl-1,3-dioxan-5-yl, 2-Methyl-cyclopentyl, 2-Methyl-cyclohexyl.

Ganz besonders bevorzugte erfindungsgemäße optisch aktive α-Fluoracrylsäureamide der Formel (I) sind das 1-Phenylethyl-, 1-Phenylpropyl-, 1-(1-Naphthyl)-ethyl-, 1-(2-Naphthyl)-ethyl-, 1-Cyclohexylethyl, Menthyl-, Carvomenthyl-, Fenchyl-, 2,2-Dimethyl-4-phenyl-1,3-dioxan-5- und 2,2-Dimethyl-4-cyclohexyl-1,3-dioxan-5-α-fluoracrylsäureamid.

Die erfindungsgemäßen optisch aktiven α-Fluoracrylsäure-Derivate der Formel (I) werden erhalten, indem man

A) optisch aktive Amine der Formel (II)

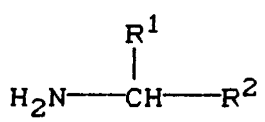 (II),

in der

$R^1$ und $R^2$ die unter der Formel (I) angegebene Bedeutung haben,

oder deren Säureadditionsprodukte

mit Fluoracrylsäure-Derivaten der Formel (III)

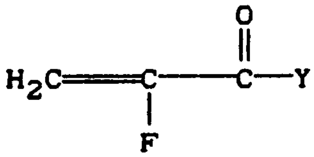

(III)

in der
Y für Fluor, Chlor oder Brom steht,
gegebenenfalls in Gegenwart eines Säurebindemittels in inerten organischen Lösungsmitteln umsetzt,
oder
B) Verbindungen der Formel (II) mit einer Fluorverbindung der Formel

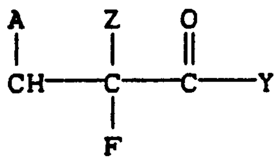

(IV)

in der

| | |
|---|---|
| Y | die oben angegebene Bedeutung hat, und |
| A und Z | jeweils für Wasserstoff, Fluor, Chlor oder Brom stehen, wobei A und Z nie gleichzeitig Wasserstoff bedeuten, |

umsetzt,
so daß die Fluoracryloyl-Verbindung (III) durch AZ-Abspaltung freigesetzt werden kann,
wobei AZ bevorzugt HF, HCl, HBr, $Br_2$ oder $Cl_2$ bedeutet.

α-Fluoracrylsäure-Derivate der Formel (III) oder deren Vorstufen (IV) können nach an sich bekannten Verfahren hergestellt werden [Zh. Org. Khim. 28, 1173 (1983)] und gegebenenfalls auch als Säureanhydride eingesetzt werden.

Die als Ausgangsverbindungen verwendeten optisch aktiven Amine der Formel (II) sind bekannt oder können nach an sich bekannten Verfahren hergestellt werden (siehe Tetrahedron Lett. 1989, 317).

Geeignete Säureadditionsverbindungen der als Ausgangsverbindungen zu verwendenden Amine sind Salze dieser Amine mit anorganischen oder organischen Säuren. Bevorzugt sind Mineralsäuren wie Salzsäure, Bromwasserstoffsäure, Schwefelsäure, Phosphorsäure oder organische Säuren wie Essigsäure, Methan-, Ethan-, Benzol- oder Toluolsulfonsäure.

Als Lösungsmittel eignen sich alle unter den Reaktionsbedingungen inerten organischen Lösungsmittel. Bevorzugt sind Kohlenwasserstoffe wie Benzol, Toluol, Xylol oder Erdölfraktionen, oder Halogenkohlenwasserstoffe wie Di-, Tri- oder Tetrachlormethan, Dichlorethan oder Trichlorethylen oder Ether wie tert.-Butylmethylether oder tert.-Amyl-ethylether.

Als Säurebindemittel kommen vor allem die üblichen anorganischen oder organischen Basen in Betracht; bevorzugt werden Alkali- oder Erdalkalihydroxide wie Natrium-, Kalium-, Lithium-, Calcium- oder Barium-hydroxid, Alkali- oder Erdalkalicarbonate wie Natrium- oder Kaliumcarbonat oder Natriumhydrogencarbonat, Alkalialkoholate wie Natriummethylat, -ethylat, Kaliummethylat, -ethylat, -t-butylat oder Amine wie Triethylamin oder Pyridin, verwendet.

Die Umsetzung der α-Fluoracrylsäure-Derivate der Formel (III) mit den Amin-Derivaten der Formel (II) wird bevorzugt bei Temperaturen von -78 bis +100°C, insbesondere von -20°C bis +60°C vorgenommen.

Die Erfindung betrifft auch die durch Polymerisation bzw. Copolymerisation der optisch aktiven α-Fluoracrylsäure-Derivateder Formel (I) erhältlichen optisch aktiven Polymere und Copolymere, die mindestens 40 Mol-%, vorzugsweise mindestens 50 Mol-% Struktureinheiten der Formel (V)

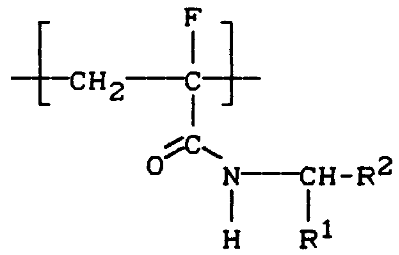

(V)

enthalten,
in der $R^1$ und $R^2$ die unter Formel (I) angegebene Bedeutung haben.

Die erfindungsgemäßen optisch aktiven Polymere der Formel (V) liegen vorzugsweise in Form von vernetzten unlöslichen oder quellbaren Perl-Polymerisaten oder in an feinteilige anorganische Trägermaterialien wie z.B. Kieselgel gebundener Form vor. Sie können auch als lineare, in geeigneten organischen Lösungsmitteln lösliche Polymere hergestellt werden. Es ist ferner möglich, verschiedene erfindungsgemäße α-Fluoracrylsäure-Derivateder Formel (I) cozupolymerisieren, sowie 0,1 bis 60, vorzugsweise 0,1 bis 20 Mol-% copolymerisierbarer, anderer Monomere in das Polymere einzubauen.

Die vernetzten Polymerisate liegen vorzugsweise in Form kleiner Teilchen (Perlen) mit 5 bis 200 µm Teilchendurchmesser vor. Sie werden z.B. durch Suspensionspolymerisation der optisch aktiven α-Fluoracrylsäure-Derivate der Formel (I) mit 0,5 bis 50 Mol-%, vorzugsweise 1 bis 30 Mol-%, besonders bevorzugt 3 bis 20 Mol-% (bezogen auf die Gesamtmenge [Mol] der eingesetzten Monomere) eines geeigneten Vernetzers in an sich bekannter Weise hergestellt.

Durch die Art und Menge der Vernetzer läßt sich der Quellungsgrad der (Perl)Polymerisate nach üblichen Methoden einstellen.

Bei der praktischen Verwendung haben sich (Perl)Polymerisate mit einem Quellungsgrad (Q) von 1.1 bis 10, bevorzugt von 2.0 bis 7,0 bewährt.

Der Quellungsgrad Q wird wie folgt bestimmt:

$$Q = \frac{\text{Polymerisatvolumen (gequollen)}}{\text{Polymerisatvolumen (ungequollen)}}$$

Als Vernetzungsmittel kommen Verbindungen in Frage, die mindestens zwei polymerisierbare Vinylgruppen enthalten. Bevorzugte Vernetzungsmittel sind Alkandioldiacrylate wie 1,6-Hexandioldiacrylat, 1,4-Butandioldiacrylat, 1,3-Propandioldiacrylat oder 1,2-Ethylenglykoldiacrylat, oder Alkandiolmethacrylate wie 1,4-Butandioldimethacrylat, 1,3-Propandioldimethacrylat oder 1,2-Ethylenglykoldimethacrylat, aromatische Divinylverbindungen wie beispielsweise Divinylbenzol, Divinylchlorbenzol oder Divinyltoluol, Dicarbonsäurevinylester wie Adipinsäuredivinylester, Benzoldicarbonsäuredivinylester, Terephthalsäuredivinylester, N,N'-Alkylendiacrylamide wie N,N'-Methylendiacrylamid, N,N'-Ethylendiacrylamid, N,N'-Methylendimethacrylamidoder N,N'-Ethylendimethacrylamid.

Als Radikalbildner kommen die üblichen Radikalbildner in Frage. Bevorzugt sind Peroxide wie beispielsweise Dibenzoylperoxid, Dilauroylperoxid oder Di-orthotolylperoxid oder Azoverbindungen wie beispielsweise Azobisisobuttersäurenitril (AIBN). Auch Gemische verschiedener Radikalbildner sind verwendbar.

Die Polymerisationskomponenten werden in einem nicht mit Wasser mischbaren organischen Lösungsmittel, vorzugsweise einem aliphatischen oder aromatischen Kohlenwasserstoff wie Hexan, Heptan, Isododecan, Benzol oder Toluol, einem Halogenkohlenwasserstoff wie Di-, Tri-, Tetrachlormethan oder 1,2-Dichlorethan oder einem Ester wie Essigsäureethylester bzw. Essigsäurebutylester gelöst.

Die organische Phase wird mit Hilfe eines wirksamen Rührers in der wäßrigen Lösung eines Schutzkolloids, bevorzugt in einer wäßrigen Lösung von Polyvinylalkohol, Polyvinylpyrrolidon oder eines Copolymers aus Methacrylsäure und Methylmethacrylat, gleichmäßig verteilt. Je Gewichtsteil organische Phase verwendet man etwa 1 bis 20, bevorzugt 2 bis 10 Gew.-Teile wäßrige Phase. Das Polymerisationsgemisch wird unter Rühren in einer Inertgasatmosphäre, vorzugsweise unter Stickstoff, auf Temperaturen von 30 °C bis 100 °C, vorzugsweise 40 °C bis 80 °C erhitzt. Die Polymerisationsdauer beträgt zwischen 2 und 24,

bevorzugt 4 und 12 Stunden. Das auf diese Weise erhaltene Copolymerisat wird durch Filtration von der flüssigen Phase getrennt, durch gründliches Waschen mit Wasser und mit organischen Lösungsmitteln wie Methanol, Ethanol, Benzol, Toluol, Di-, Trichlormethan oder Aceton gereinigt und anschließend getrocknet.

Insbesondere für analytische Anwendungen werden die erfindungsgemäßen optisch aktiven Polymere vorzugsweise in an feinteilige anorganische Träger gebundener Form eingesetzt. Die Herstellung solcher optisch aktiver Chromatographiephasen kann beispielsweise nach den in DE-A 3 706 890 beschriebenen Verfahren erfolgen.

Bevorzugt ist die Polymerisation der optisch aktiven α-Fluoracrylsäure-Derivate der Formel (I) in Gegenwart von Kieselgel-Diolphasen, die mit (Meth)acrylsäure verestert wurden. Die Polymerisation kann dabei in Abwesenheit von Lösungsmitteln oder in Gegenwart von Lösungsmitteln oder von Fällungsmitteln für die Poly-α-fluoracrylamide durchgeführt werden. Als Initiatoren können die zur Herstellung der Perlpolymerisate verwendeten Radikalbildner ebenfalls eingesetzt werden.

Die polymermodifizierten Kieselgele enthalten vorzugsweise 1 bis 40 Gew.-%, insbesondere 5 bis 30 Gew.-% an optisch aktivem Polymer (V), bezogen auf das Gesamtgewicht. Sie werden intensiv mit Lösungsmitteln für das Polymere gewaschen und im Vakuum getrocknet.

Selbstverständlich können auch hier Gemische von zwei oder mehreren der erfindungsgemäßen α-Fluoracrylsäure-Derivate, gegebenenfalls auch mit weiteren copolymerisierbaren Monomeren eingesetzt werden.

Die Erfindung betrifft ferner die Verwendung der erfindungsgemäßen Polymere als solche oder in vernetzter bzw. an Kieselgel gebundener Form zur chromatographischen Trennung von racemischen Gemischen in die optischen Antipoden. Gut trennbare Racemate sind beispielsweise Oxazepam, Binaphthol, 1,1'-Binaphthyl-2,2'-dicarbonsäure, Thalidomid, Chlormezanon, Hexahydrocarbazolderivate wie z.B. 3-r-(Benzol-sulfonamido)-9-(2-carboxyethyl)-1,2,3,4,4a,9a-hexahydrocarbazol und Dihydropyridine wie z.B. 1,4-Dihydro-2,6-dimethyl-4-(2-trifluormethylphenyl)-pyridin-3,5-dicarbonsäure-5-ethylester.

Die Zusammensetzung des Fließmittels kann je nach Art und Eigenschaft des zu trennenden Racemates in üblicher Weise ausgewählt und optimiert werden. Die an Kieselgel gebundenen erfindungsgemäßen Poly-α-fluoracrylsäurederivate können für chromatographische Racemattrennungen unter HPLC-Bedingungen eingesetzt werden.

Die Fähigkeit der Polymerisate zur Racemattrennung wird durch die Kapazitätsverhältnisse ($k'_{1(2)}$-Werte) für die beiden Enantiomere (1) und (2) und den daraus resultierenden Enantioselektivitätswert α ausgedrückt. Diese chromatographischen Parameter sind wie folgt definiert:

$$\text{Kapazitätsverhältnis } k_{1(2)} = \frac{t_{1(2)} - t_o}{t_o}$$

$$\text{Enantioselektivität } \alpha = \frac{k'_2}{k'_1}$$

$t_o$ = Totzeit der Säule

$t_{1(2)}$ = Retentionszeit des zuerst eluierten Enantiomers 1 bzw. des später eluierten Enantiomers 2

Die präparative Trennung von racemischen Gemischen in ihre optischen Antipoden unter Verwendung der erfindungsgemäßen Polymerisate wird vorzugsweise säulenchromatographisch vorgenommen. Besonders vorteilhaft ist es hierfür, die chromatographische Trennung mit Perlpolymerisaten einer bestimmten Korngrößenverteilung vorzunehmen; gute Trennleistungen werden mit Perlpolymerisaten einer Korngrößenverteilung von 5 bis 200 µm, bevorzugt 15 bis 100 µm erhalten.

Die Arbeitsmethodik der säulenchromatographischen Trennung ist bekannt. Üblicherweise wird das Polymerisat im Fließmittel suspendiert und die Suspension in eine Glassäule gefüllt. Nach Ablaufen des Fließmittels wird das zu trennende Racemat, gelöst in möglichst wenig Fließmittel, auf die Säule aufgebracht. Dann wird mit Fließmittel eluiert und die Enantiomeren im Eluat photometrisch und/oder polarimetrisch mittels geeigneter Durchflußzellen detektiert.

Als Fließmittel werden übliche organische Lösungsmittel bzw. Lösungsmittelgemische verwendet, die das als Adsorbens eingesetzte Polymerisat anquellen und das zu trennende Racemat lösen. Beispielsweise

seien genannt: Kohlenwasserstoffe wie Benzol, Toluol oder Xylol, Ether wie Diethylether, Dioxan oder Tetrahydrofuran, Halogenkohlenwasserstoffe wie Di- oder Trichlormethan, Aceton, Acetonitril oder Essigester, Alkohole wie Methanol, Ethanol, n-Propanol, Isopropanol oder n-Butanol, oder aber Gemische der genannten Lösungsmittel. Als besonders geeignet haben sich Mischungen aus Toluol mit Tetrahydrofuran, Dioxan oder Isopropanol erwiesen.

Beispiele

1. Herstellung der α-Fluoracrylsäureamide (Monomere)

1.1 N-Fluoracryloyl-S-1-phenethylamin

Zu einer Lösung von 26,6 g (0,22 Mol) S-Phenethylamin und 22,2 g (0,22 Mol) Triethylamin in 400 ml abs. Dichlormethan tropfte man bei -10° C eine Lösung von 20,8 g (0,22 Mol) α-Fluoracrylsäurechlorid in 20 ml abs. Dichlormethan. Man ließ 2 Stunden ohne Kühlbad nachrühren, wusch erst mit Wasser, dann mit 1 N HCl (2 x) und trocknete den organischen Extrakt über Magnesiumsulfat. Nach Evaporieren des Solvens verblieben 41,8 g Feststoff vom Fp. 87° C. Umkristallisation aus Petrolether/Toluol 2:1 ergab 29,7 g (70 % d. Th.) reines Fluoracrylamid vom Fp. 93° C.
Drehwert (c = 1, $CHCl_3$) -175,7°

1.2 N-Fluoracryloyl-S-cyclohexylethylamin

Analog erhielt man aus 26,16 g (0,206 Mol) S-Cyclohexylethylamin 38,6 g Rohprodukt. Kristallisation aus Petrolether/Toluol 15:1 ergab 23 g (58 % d. Th.) Fluoracrylamid vom Fp. 86° C.
Drehwert (c = 1, $CHCl_3$) -37,9°

1.3 N-Fluoracryloyl-d-menthylamin

Durch Einsatz von 31 g (0,2 Mol) d-Menthylamin wurden 37,5 g Rohprodukt isoliert, das durch Chromatographie an Silicagel mit Ether/Petrolether 1:2 gereinigt wurde.
Ausbeute: 24 g (52 %), Fp.: 36° C
Drehwert (c = 1, $CHCl_3$) +48,6°

1.4 N-Fluoracryloyl-(4S,5S)-5-amino-2,2-dimethyl-4-phenyl-1,3-dioxan

Führt man die Reaktion mit 41 g (0,2 Mol) (4S,5S)-5-Amino-2,2-dimethyl-4-phenyl-1,3-dioxan durch, so erhält man 51 g Rohprodukt. Reinigung an Silicagel mit Ether/Petrolether 7:2 ergab 29,4 g Fluoracrylamid vom Fp. 94° C.
Drehwert (c = 1, $CHCl_3$) +30,5°

2. Polymerisation der N-α-Fluoracrylsäureamide

2.1 Herstellung in an Kieselgel gebundener Form

a) 25 g mit 1,2-Diolgruppen modifiziertes Kieselgel (mittlere Korngröße: 5 µm, Analysenwerte C: 7,7 %, H: 1,5 %) werden unter Feuchtigkeitsausschluß und unter Stickstoff in 500 ml Dioxan suspendiert. Die Suspension wird mit 16 ml Methacrylsäureanhydrid und 12,5 ml Triethylamin versetzt. Die Mischung wird 1 Stunde bei Raumtemperatur gerührt und 24 Stunden bei Raumtemperatur aufbewahrt. Dann wird das Kieselgel über eine Glasfritte (G4) abgesaugt, 3 x mit je 500 ml Dioxan 30 Minuten lang verrührt und zwischendurch gut trockengesaugt. Das durch Methacryloyl-Gruppen modifizierte Kieselgel wird bei Raumtemperatur im Vakuum <0,005 at getrocknet.
Ausbeute: 24,8 g
Elementaranalyse: C 9,2 %; H 1,7 %
b) In einem mit Rückflußkühler und Magnetrührer versehenen 100 ml-Rundkolben werden 3 g des mit Methacryloylgruppen modifizierten Kieselgels, dessen Herstellung unter a) beschrieben ist, 6,0 g optisch aktives N-α-Fluoracryloyl-amin und 60 mg Azobisisobutyronitril in 25 ml trockenem Toluol gelöst bzw. suspendiert. Die Apparatur wird durch dreimaliges Evakuieren und mit Stickstoffüllen von Luft befreit und abschließend mit Stickstoff befüllt. Das Polymerisationsgemisch wird 1 Stunde bei Raumtemperatur

gerührt und dann rasch auf 80 °C erhitzt. Nach 45-minütigem Rühren bei 80 °C werden 200 mg 2,6-Di-tert.-butyl-4-methylphenol zugesetzt und das Reaktionsgemisch rasch abgekühlt. Das Kieselgel wird über eine Glasfritte (G4) abgesaugt, mit Toluol gewaschen und 2 x mit je 50 ml Chloroform, 1 x mit 50 ml Toluol und 1 x mit 50 ml Isopropanol je 30 Minuten verrührt und zwischendurch abgesaugt. Das Kieselgel wird abschließend bei Raumtemperatur im Vakuum <0,005 at getrocknet. In der nachstehenden Tabelle 1 sind die auf das modifizierte Kieselgel aufpolymerisierten N-α-Fluoracryloyl-amine, die Ausbeuten an optisch aktive Verbindungen enthaltendem Kieselgel, dessen Stickstoffgehalt und dessen Gehalt an gebundenem Polymer zusammengestellt.

**Tabelle 1**

| Beispiel Nr. | N-α-Fluoracryloylamin gemäß Beispiel | Ausbeute (g) | (N-Gehalt) (%) | Gehalt des Kieselgels an gebundemem Polymerisat (Gew.-%) |
|---|---|---|---|---|
| 2.5 | 1 | 3,4 | 1,3 | 17,9 |
| 2.6 | 2 | 3,3 | 1,2 | 17,1 |
| 2.7 | 3 | 3,5 | 1,0 | 16,2 |
| 2.8 | 4 | 3,2 | 0,8 | 16,0 |

3. Herstellung in Form von Perlpolymerisaten

Die Lösung von 13,5 g optisch aktiven N-α-Fluoracryloylamin, 1,5 g Ethylenglykoldimethacrylat und 0,3 g Azobisisobutyronitril in 37,5 g Trichlormethan wird unter Rühren (350 bis 500 U/min) in einer Lösung von 3 g Polyvinylalkohol in 130 ml entmineralisiertem Wasser dispergiert. Die Apparatur wird mehrmals evakuiert und mit Stickstoff gefüllt. Das Polymerisationsgemisch wird unter Stickstoff zunächst 30 Minuten

bei Raumtemperatur und anschließend 16 Stunden bei 55 °C (Innentemperatur) gerührt. Das Polymerisationsgemisch wird dann in 2 bis 3 l Wasser eingerührt und die flüssige Phase nach dem Absitzen des Perlpolymerisates dekantiert. Das Perlpolymerisat wird durch 3 bis 4-maliges Suspendieren in Wasser und Abdekantieren der flüssigen Phase vom Feinkorn (Polymerisat mit einer Korngröße von $<10\ \mu m$) befreit und nach intensivem Waschen mit Aceton bei 60 °C bis zur Gewichtskonstanz getrocknet.

In der nachstehenden Tabelle 2 sind die für die Polymerisation verwendeten N-α-Fluoracryloyl-amine, die Rührgeschwindigkeit, bei der die Polymerisation durchgeführt wurde, die Ausbeuten, in denen die Polymerisate erhalten wurden, deren Korngröße und das Volumen der erhaltenen Perlpolymerisate in trockenem (Vs) und gequollenem (Vq) Zustand (Quellmittel: Toluol = T bzw. Toluol/THF = 3:2 v/v-Gemisch = T/T) zusammengestellt.

**Tabelle 2**

| Beispiel Nr. | N-Fluoracryloyl-amin gemäß Beispiel | Rührgeschwindigkeit (U/min) | Ausbeute an Perlen (g) | Korngröße der Perlen (µm) | $V_s$ (ml/g) | $V_q$ (ml/g) | Quellmittel |
|---|---|---|---|---|---|---|---|
| 3.9 | 1 | 450 | 11,1 | 20-90 | 2,5 | 10,0 | T/T |
| 3.10 | 2 | 400 | 11,3 | 30-110 | 1,6 | 5,8 | T |
| 3.11 | 3 | 350 | 10,9 | 30-120 | 2,0 | 5,5 | T |
| 3.12 | 4 | 350 | 10,4 | 20-110 | 1,5 | 4,8 | T |

Verwendung der optisch aktiven Polymerisate der α-Fluoracrylsäure-Derivate als Adsorbentien für die Racemattrennung

Für die chromatographischen Trennungen wurden folgende Testracemate verwendet:

Racemat Nr. 1: Oxazepam
Racemat Nr. 2: Binaphthol
Racemat Nr. 3: Chlormezanon

Racemat Nr. 4: 1,1'-Binaphthyl-2,2'-dicarbonsäure
Racemat Nr. 5: 1,4-Dihydro-2,6-dimethyl-5-nitro-4-(2-trifluormethylphenyl)-pyridin-3-carbonsäureethylester
Racemat Nr. 6: 3-r-(4-Fluorphenylsulfonamido)-9-(2-carboxyethyl)-1,2,3,4,4a, 9a-hexahydrocarbazol

Die bei der chromatographischen Trennung der verschiedenen Testracemate 1 bis 6 mit Hilfe der erfindungsgemäßen Adsorbentien erhaltenen Ergebnisse (Enantioselektivität $\alpha$ und Kapazitätsverhältnis $k_1$') und die verwendeten Elutionsmittel sind in der folgenden Tabelle zusammengestellt.

Die an Kieselgel gebundenen Polymerisate wurden in Stahlsäulen (Innendurchmesser: 4 mm; Länge: 25 cm) eingesetzt. Eluiert wurde mit n-Heptan-Tetrahydrofuran-Gemischen: a) 3:2 v/v; b) 1:1 v/v.

Die überraschende Trennwirkung der erfindungsgemäßen polymeren Fluoracrylamide wird durch direkten Vergleich mit den entsprechenden polymeren (Meth)Acrylamiden offensichtlich, die den gleichen optisch aktiven Rest enthalten.

Die Vergleiche wurden mit den an Kieselgel fixierten chiralen Polymeren durchgeführt.

So läßt sich beispielsweise der Tranquilizer Oxazepam (Testracemat 1) am erfindungsgemäßen Polyfluoracrylamid des optisch aktiven 1-Phenethylamins (Adsorbens 5) fast vollständig in die Enantiomere trennen, während weder das polymere Acryl- noch das Methacrylamid des 1-Phenethylamins eine Trennwirkung zeigen.

In einem anderen Fall zeigt obiges Polyfluoracrylamid (Adsorbens 5) für den als Racemat vorliegenden Thromboxan-Antagonisten 3-r-(Benzolsulfonamido)-9-(2-carboxyethyl)-1,2,3,4,4a,9a-hexahydrocarbazol (Testracemat 6) eine Trennwirkung, während das entsprechende Polyacrylamid keinerlei Antrennung erkennen läßt.

In ähnlicher Weise trennt das erfindungsgemäße Polyfluoracrylamid des optisch aktiven 1-Cyclohexylethylamins (Adsorbens 6) Oxazepam (Testracemat 1) fast vollständig in die Enantiomere, während das entsprechende Polymethacrylamid nur eine sehr schlechte Trennwirkung für das Oxazepam-Racemat aufweist.

Das erfindungsgemäße polymere Fluoracrylamid des optisch aktiven Menthylamins (Adsorbens 7) zeigt eine deutliche Trennwirkung für 6,6'-Dimethylbiphenyl-2,2'-dicarbonsäure (Testracemat 4), während das entsprechende polymere Methacrylamid nur eine geringe Aktivierung für dieses Racemat zeigt (Beispiel 4).

<u>Tabelle 3</u> - Kieselgeltrennungen

| Adsorbens gem. Bsp. | Testracemat | Enantioselektivität $\alpha$ | Kapazitätsverhältnis $k_1$ | |
|---|---|---|---|---|
| 5 | 1 | 1,21 | 2,59 | b |
| | 2 | 1,11 | 4,95 | a |
| | 6 | 1,15 | 1,88 | b |
| 6 | 1 | 1,17 | 2,39 | b |
| 7 | 1 | 1,25 | 3,00 | a |
| | 4 | 1,23 | 1,59 | a |
| | 5 | 1,12 | 0,92 | a |
| 8 | 3 | 1,10 | 2,36 | a |

**Patentansprüche**

1. Optisch aktive α-Fluoracrylsäureamide der Formel (I)

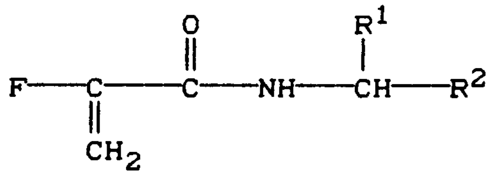

(I)

in welcher

$R^1$ für einen Phenyl-, Naphthyl-, Pyridyl- oder $C_3$-$C_{10}$-Cycloalkylrest steht, der gegebenenfalls substituiert ist durch Halogen, $C_1$-$C_4$-Alkoxy, $NR^3COR^4$, Dioxyalkylen mit 1 bis 2 C-Atomen, Alkyl oder Cycloalkyl mit jeweils bis zu 10 C-Atomen, wobei

$R^3$ für Wasserstoff oder $C_1$-$C_4$-Alkyl steht und

$R^4$ für Alkyl oder Alkoxy mit jeweils 1 bis 6 C-Atomen, für Phenyl oder Benzoxy steht,

$R^2$ für Alkyl oder Cycloalkyl mit jeweils bis zu 8 C-Atomen, gegebenenfalls substituiert durch Halogen oder durch $C_1$-$C_4$-Alkoxy steht,

oder

$R^1$ und $R^2$ gemeinsam für einen cyclischen Rest aus der Gruppe Terpenyl, 8-Alkyl($C_1$-$C_4$)-menthyl, 8-Aryl($C_6$-$C_{10}$)-menthyl, Tetrahydropyryl, 2,2-Dialkyl($C_1$-$C_4$)-4-aryl-($C_6$-$C_{10}$)-1,3-dioxan-5-yl, 2,2-Dialkyl-($C_1$-$C_4$)-4-cycloalkyl($C_3$-$C_8$)-1,3-dioxan-5-yl, Spiro-cycloalkyl($C_3$-$C_8$)-2'-4-aryl-($C_6$-$C_{10}$)-1,3-dioxan-5-yl, Spiro-cycloalkyl($C_3$-$C_8$)-2'-4-cycloalkyl($C_3$-$C_8$)-1,3-dioxan-5-yl, $C_1$-$C_4$-alkylsubstituierter $C_3$-$C_8$-Cycloalkylrest steht.

2. Verbindungen der Formel (I) gemäß Anspruch 1, in welcher

$R^1$ für Naphthyl, Pyridyl oder Phenyl steht, welches gegebenenfalls ein- oder zweifach substituiert ist durch gleiche oder verschiedene Substituenten aus der Gruppe Fluor, Chlor, Alkyl mit 1 bis 4 C-Atomen, Alkoxy mit 1 bis 4 C-Atomen, Cycloalkyl mit 3 bis 6 C-Atomen, Methylendioxy, Ethylendioxy, Acetamido, Benzamido, Carbomethoxyamino, Carboethoxyamino, Carbobenzoxyamino oder für einen Cycloalkylrest mit 3 bis 7 C-Atomen oder für einen Decahydronaphthylrest steht,

$R^2$ für Alkyl oder Cycloalkyl mit jeweils bis zu 8 C-Atomen steht, wobei der Alkylrest gegebenenfalls durch Fluor, Chlor oder Alkoxy mit 1 bis 2 C-Atomen substituiert ist,

oder

$R^1$ und $R^2$ gemeinsam für einen Rest stehen aus der Gruppe Menthyl, Neomenthyl, Isomenthyl, Neoisomenthyl, Carvomenthyl, Bornyl, Fenchyl, Pinanyl, Isopinocampheyl, 8-Methylmenthyl, 8-Ethyl-menthyl, 8-n-Propyl-menthyl, 8-i-Propylmenthyl, 8-n-Butylmenthyl, 8-i-Butylmenthyl, 8-s-Butylmenthyl, 8-t-Butylmenthyl, 8-Phenylmenthyl, 8-Naphthylmenthyl, Tetrahydropyryl, 2,2-Dimethyl-4-phenyl-1,3-dioxan-5-yl, 2,2-Dimethyl-4-naphthyl-1,3-dioxan-5-yl, 2,2-Diethyl-4-phenyl-1,3-dioxan-5-yl, 2,2-Diethyl-4-naphthyl-1,3-dioxan-5-yl, 2,2-Dipropyl-4-phenyl-1,3-dioxan-5-yl, 2,2-Dipropyl-4-naphthyl-1,3-dioxan-5-yl, 2,2-Dibutyl-4-phenyl-1,3-dioxan-5-yl, 2,2-Dibutyl-4-naphthyl-1,3-dioxan-5-yl analog mit 2,2-Dibutyl-4-cyclohexyl-1,3-dioxan-5-yl, Spiro-cyclopentyl-4,2'-4-phenyl-1,3-dioxan-5-yl, Spiro-cyclopentyl-4,2'-4-naphthyl-1,3-dioxan-5-yl, Spiro-cyclopentyl-4,2'-4-cyclohexyl-1,3-dioxan-5-yl, Spiro-cyclohexyl-5,2'-4-phenyl-1,3-dioxan-5-yl, Spiro-cyclohexyl-5,2'-4-naphthyl-1,3-dioxan-5-yl, Spiro-cyclohexyl-5,2'-4-cyclohexyl-1,3-dioxan-5-yl, 2-Methyl-cyclopentyl, 2-Ethyl-cyclopentyl, 2-Propyl-cyclopentyl, 2-i-Propyl-cyclopentyl, 2-n-Butyl-cyclopentyl, 2-i-Butyl-cyclopentyl, 2-s-Butyl-cyclopentyl, 2-t-Butyl-cyclopentyl, 2-Methyl-cyclohexyl, 2-Ethyl-cyclohexyl, 2-Propyl-cyclohexyl, 2-i-Propyl-cyclohexyl, 2-n-Butyl-cyclohexyl, 2-i-Butyl-cyclohexyl, 2-s-Butyl-cyclohexyl, 2-t-Butyl-cyclohexyl.

**3.** Verbindungen der allgemeinen Formel (I) gemäß Anspruch 1, in welcher

$R^1$ für Phenyl steht, welches gegebenenfalls einfach substituiert ist durch Fluor, Chlor, Methyl, Methoxy, Nitro, Acetamido, Benzamido, Carbomethoxyamino, Carboethoxyamino, Ethyl, Propyl oder Butyl oder für 1-Naphthyl, 2-Naphthyl, 2-Pyridyl, 3-Pyridyl, 4-Pyridyl, Decahydronaphthyl oder Cycloalkyl mit 3 bis 6 C-Atomen steht,

$R^2$ für Alkyl mit 1 bis 4 C-Atomen, welches gegebenenfalls durch Fluor, Chlor, Methoxy oder Ethoxy substituiert ist oder für Cyclopentyl oder Cyclohexyl steht oder

$R^1$ und $R^2$ gemeinsam für einen Rest stehen aus der Gruppe Menthyl, Neomenthyl, Isomenthyl, Neoisomenthyl, Carvomenthyl, Bornyl, Fenchyl, Pinanyl, Isopinocampheyl, 8-Methylmenthyl, 8-Phenylmenthyl, Tetrahydropyryl, 2,2-Dimethyl-4-phenyl-1,3-dioxan-5-yl, 2,2-Diethyl-4-phenyl-1,3-dioxan-5-yl, 2,2-Diethyl-4-phenyl-1,3-dioxan-5-yl, 2,2-Dipropyl-4-phenyl-1,3-dioxan-5-yl, 2,2-Dibutyl-4-phenyl-1,3-dioxan-5-yl, 2,2-Dimethyl-4-cyclohexyl-1,3-dioxan-5-yl, 2,2-Diethyl-4-cyclohexyl-1,3-dioxan-5-yl, 2,2-Dipropyl-4-cyclohexyl-1,3-dioxan-5-yl, 2,2-Dibutyl-4-cyclohexyl-1,3-dioxan-5-yl, Spiro-cyclopentyl-4,2'-4-phenyl-1,3-dioxan-5-yl, Spiro-cyclopentyl-4,2'-4-cyclohexyl-1,3-dioxan-5-yl, Spiro-cyclohexyl-5,2'-4-phenyl-1,3-dioxan-5-yl, Spiro-cyclohexyl-5,2'-4-cyclohexyl-1,3-dioxan-5-yl, 2-Methyl-cyclopentyl, 2-Methyl-cyclohexyl.

**4.** Verfahren zur Herstellung von optisch aktiven α-Fluoracrylsäureamiden der allgemeinen Formel (I)

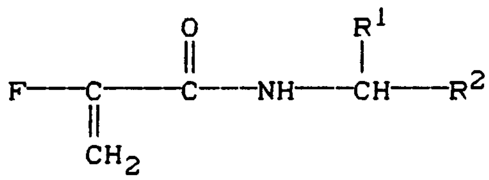

(I)

in welcher

$R^1$ für einen Phenyl-, Naphthyl-, Pyridyl- oder $C_3$-$C_{10}$-Cycloalkylrest steht, der gegebenenfalls substituiert ist durch Halogen, Nitro, $C_1$-$C_4$-Alkoxy, $NR^3COR^4$, Dioxyalkylen mit 1 bis 2 C-Atomen, Alkyl oder Cycloalkyl mit jeweils bis zu 10 C-Atomen, wobei

$R^3$ für Wasserstoff oder $C_1$-$C_4$-Alkyl steht und

$R^4$ für Alkyl oder Alkoxy mit jeweils 1 bis 6 C-Atomen, für Phenyl oder Benzoxy steht,

$R^2$ für Alkyl oder Cycloalkyl mit jeweils bis zu 8 C-Atomen, gegebenenfalls substituiert durch Halogen oder durch $C_1$-$C_4$-Alkoxy steht,

oder

$R^1$ und $R^2$ gemeinsam für einen cyclischen Rest aus der Gruppe Terpenyl, 8-Alkyl($C_1$-$C_4$)-menthyl, 8-Aryl($C_6$-$C_{10}$)-menthyl, Tetrahydropyryl, 2,2-Dialkyl($C_1$-$C_4$)-4-aryl($C_6$-$C_{10}$)-1,3-dioxan-5-yl, 2,2-Dialkyl($C_1$-$C_4$)-4-cycloalkyl($C_3$-$C_8$)-1,3-dioxan-5-yl, Spiro-cycloalkyl($C_3$-$C_8$)-2'-4-aryl($C_6$-$C_{10}$)-1,3-dioxan-5-yl, Spiro-cycloalkyl($C_3$-$C_8$)-2'-4-cycloalkyl($C_3$-$C_8$)-1,3-dioxan-5-yl, $C_1$-$C_4$-alkylsubstituierter $C_3$-$C_8$-Cycloalkylrest steht,

dadurch gekennzeichnet, daß man optisch aktive Amine der Formel (II)

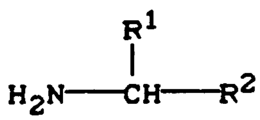

(II),

in der

$R^1$ und $R^2$ die unter der Formel (I) angegebene Bedeutung haben,

oder deren Säureadditionsprodukte

mit Fluoracrylsäure-Derivaten der Formel (III)

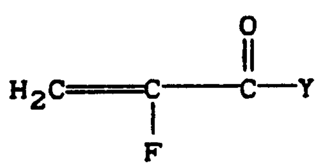

(III)

in der

Y für Fluor, Chlor oder Brom steht,

gegebenenfalls in Gegenwart eines Säurebindemittels in inerten organischen Lösungsmitteln umsetzt,

oder

B) Verbindungen der Formel (II) mit einer Fluorverbindung der Formel

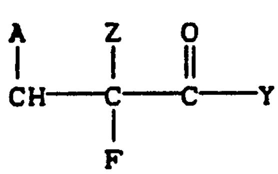

(IV)

in der

Y die oben angegebene Bedeutung hat, und

A und Z jeweils für Wasserstoff, Fluor, Chlor oder Brom stehen, wobei A und Z nie gleichzeitig Wasserstoff bedeuten,

umsetzt,

so daß die Fluoracryl-Verbindung (III) durch AZ-Abspaltung freigesetzt werden kann.

5. Optisch aktive Polymere enthaltend mindestens 40 Mol-% von Struktureinheiten der Formel (V)

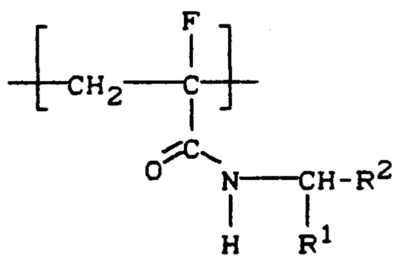

(V)

in welcher

$R^1$ und $R^2$ die in Anspruch 1 angegebene Bedeutung haben.

6. Optisch aktive Polymere gemäß Anspruch 5, dadurch gekennzeichnet, daß sie in Form von vernetzten unlöslichen oder quellbaren Perlpolymerisaten oder in an feinteilige anorganische Trägermaterialien gebundener Form vorliegen.

7. Verfahren zur Herstellung von vernetzten Perlpolymerisaten gemäß Anspruch 6, dadurch gekennzeichnet, daß man die N-$\alpha$-Fluoracrylsäure-Derivate der allgemeinen Formel (I) mit 0,5 bis 50 Mol-% eines Vernetzers durch Suspensionspolymerisation, gegebenenfalls in Anwesenheit von Radikalbildnern und inerten organischen Lösungsmitteln herstellt.

8. Verfahren zur Herstellung von an anorganische Trägermaterialien gebundenen optisch aktiven Polymere gemäß Anspruch 6, dadurch gekennzeichnet, daß man Verbindungen der allgemeinen Formel (I) gemäß Anspruch 1 in Gegenwart von gegebenenfalls (Meth)-acrylsäure-substituierten Kieselgel-Diolphasen gegebenenfalls in Anwesenheit von Radikalbildnern und gegebenenfalls in Anwesenheit von inerten organischen Lösungsmitteln verestert.

9. Verwendung von Polymeren gemäß Anspruch 7 zur Trennung von optischen Isomeren.

Europäisches Patentamt

# EUROPÄISCHER RECHERCHENBERICHT

Nummer der Anmeldung

**EP 91 11 0141**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 218 089 (BAYER)<br>* Insgesamt *<br>– – – | 1,5,9 | C 07 C 233/09<br>C 07 B 57/00<br>C 08 F 20/54<br>C 07 D 319/06<br>B 01 J 20/32 |
| A | EP-A-0 249 078 (MERCK)<br>* Insgesamt *<br>– – – | 1,5,9 | |
| A | EP-A-0 279 331 (DAIKIN INDS.)<br>* Insgesamt *<br>– – – | 5 | |
| A | PATENT ABSTRACTS OF JAPAN, Band 8, Nr. 122 (C-227)[1559], 8. Juni 1984;<br>& JP-A-59 33 307 (NIPPON GOSEI GOMU K.K.) 23-02-1984<br>* Zusammenfassung *<br>– – – | 1,5,9 | |
| A | PATENT ABSTRACTS OF JAPAN, Band 12. Nr. 139 (C-491)[2986], 27. April 1988;<br>& JP-A-62 255 548 (DAIKIN IND., LTD) 07-11-1987<br>* Zusammenfassung * & GENERIC DARC ONLINE DARSTELLUNG VON CHEMICAL ABSTRACTS, Registry Nr. 114299-48-8<br>– – – – – | 1 | |

**RECHERCHIERTE SACHGEBIETE (Int. Cl.5)**

C 07 C 233/00
C 07 B 57/00
C 08 F 20/00
C 07 D 319/00

Der vorliegende Recherchenbericht wurde für alle Patentansprüche erstellt

| Recherchenort | Abschlußdatum der Recherche | Prüfer |
|---|---|---|
| Den Haag | 02 Oktober 91 | WELLS A.G. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument